# EUROPEAN PATENT SPECIFICATION

(11) **EP 4 315 003 B1**
(45) Date of publication and mention of the grant of the patent: **04.11.2026**
(21) Application number: 22719912.2
(22) Date of filing: 31.03.2022
(51) Int. Cl.: G06F 3/01, G06F 3/0346, A61B 3/113

(54) **EYEWEAR FOR DETERMINING AN EYE DIRECTION**
BRILLE ZUR BESTIMMUNG EINER AUGENRICHTUNG
LUNETTES POUR DÉTERMINER UNE DIRECTION D'UN OEIL

(30) Priority: 31.03.2021 EP 21305406
(43) Date of publication of application: 07.02.2024
(73) Proprietor: Essilor International, 94220 Charenton-Le-Pont (FR)
(72) Inventor: COMBIER, Jessica, 94220 CHARENTON LE PONT (FR); BOUCHIER, Aude, 94220 CHARENTON LE PONT (FR); CANO, Jean-Paul, 94220 CHARENTON-LE-PONT (FR)
(74) Representative: Plasseraud IP
(86) International application number: PCT/EP2022/058624
(87) International publication number: WO 2022/207820

(56) References cited:
- US-A1- 2021 068 652

## Description

The invention relates to an eyewear adapted for determining an eye direction.

### -- BACKGROUND OF THE INVENTION --

Determining the direction of at least one eyeball of a person is useful for many applications, for example in the ophthalmic field, the augmented reality field, etc. Generally for these applications, energy consumption and reliability of the determined eyeball direction are important issues, and the solutions proposed up to now are still not satisfactory.

Indeed, some of the existing solutions implement array-type image sensors that are arranged for capturing images of the pupil, and processing units configured for inferring the eyeball direction from the captured images. But such image sensors are energy-consuming, so that they require power sources with enough capacity to be provided. Such power sources are heavy and cumbersome, and combining them with an ophthalmic eyewear or an augmented reality equipment is difficult, resulting in devices which are unpleasant when worn for long periods.

In addition, many of the existing solutions implement light sources arranged in the vicinity of the eyeball for emitting infrared light towards the user's eye. Such light sources improve the reliability of the eyeball direction that is determined, in particular when the ambient light level is low. But these light sources further increase the energy consumption, making the issue of the weight and size of the power source even more critical.

Starting from this situation, one object of the present invention consists in providing a new device that allows determining the eyeball direction, but without the drawbacks of the existing ones.

In particular, the present invention aims at providing a device capable of determining the eyeball direction, that is simple, lightweight and easy to arrange within a face-fitting equipment.

Another object of the present invention is to provide such device that determines the eyeball direction in a reliable manner, for most of ambient light conditions.

US 2021/068652 A1 describes a known eyewear.

### -- SUMMARY OF THE INVENTION --

The invention is defined in the appended claims.

### -- BRIEF DESCRIPTION OF THE DRAWINGS --

Figure 1 is a perspective view of an eyewear according to the invention;
Figure 2a is an enlargement of one light sensor used in the eyewear of Figure 1;
Figure 2b corresponds to Figure 2a for a particular embodiment of the invention;
Figure 3a is a diagram showing steps performed by a processing unit of the eyewear of Figure 1; and
Figure 3b, 4, 5 and 6 each correspond to Figure 3a for alternative embodiments.

For clarity sake, element sizes which appear in these figures do not correspond to actual dimensions or dimension ratios. Also, same reference numbers which are indicated in different ones of these figures denote identical elements of elements with identical function.

### -- DETAILED DESCRIPTION OF THE INVENTION --

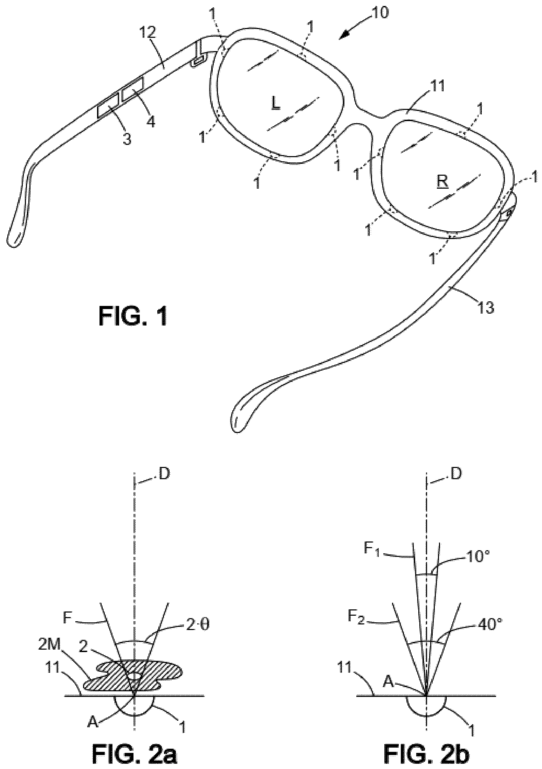

An eyewear in accordance with the present invention is generally denoted by reference number 10 in Figure 1. In the embodiment illustrated, the eyewear 10 is comprised of a spectacle equipment with face-fitting frame 11, temples 12 and 13 and left and right see-through areas denoted L and R respectively. Each one of the see-through areas L and R is dedicated to one of the eyes of a wearer of the eyewear 10. In possible embodiments of the invention, eyeglasses may be accommodated in the frame 11 at the see-through areas L and R for producing any optical function including ametropia correction, active accommodation aid, active darkening, in particular for solar protection, spectral filtering, in particular active spectral filtering, polarization filtering, in particular active polarization filtering, and information display, in particular for augmented reality applications. Anyone will also understand that the function of determining the eye direction is not limited to using eyeglasses in the see-through areas L and R, and that applications exist that require determining the eye direction for purposes not implemented within the frame 11.

A plurality of light sensors 1, for example five light sensors, are arranged in the frame 11 at the peripheral limit of each of the see-through areas L and R. Alternatively, some of the light sensors 1 may be located at the nasal bridge of the frame 11 or in the temples 12 and 13, but locations close to the see-through areas L and R are preferred for the light sensors 1 oriented towards the wearer's eye. Each light sensor 1 may be comprised of a photodiode, but other light sensor types may be used alternatively. Each light sensor may also be combined with an optical filter, and/or an electrical amplifier. Each light sensor 1 is associated with a field of view F having an aperture value and an axial direction D (see Figure 2a). According to the invention, the respective fields of view F of at least three of the light sensors 1 are oriented towards the left or right wearer's eye that corresponds to the see-through area L or R considered, with an aperture value that is comprised between 0.006 steradian and 0.22 steradian. Each field of view F has an apex A located at the corresponding light sensor 1, or more generally at the frame 11 considering some particular embodiments which will be described later. Each field-of-view cross-section increases when moving along the field-of-view axial direction D away from the apex A. The shape of each field of view F may be any in cross-section, including disk shape, square shape and elongated shape with longitudinal direction oriented in a selected manner. The shape of each field of view F may be determined by any means, including a small lens of any type, in particular refractive- or diffractive type, a hole with slanted peripheral wall or an aperture stop arranged above a photosensitive surface of the light sensor. Figure 2a shows such light sensor 1 supported by the frame 11 with its field of view F determined by a hole 2 cut in a mask 2M which is arranged above the photosensitive surface of the light sensor. The mask 2M thus determines the axial direction D of the field of view F, its cross-sectional shape and its aperture value. In the example represented, the cross-sectional shape of the field of view F is circular, so that the field of view F has a conical shape with aperture angle 2·θ. Preferably, this aperture angle 2·θ is comprised between 5° and 30°, preferably between 8° and 15°, for example equal to 10°. The field of view F is oriented towards the wearer's face, so as to contain at least part of the wearer's eye. In such embodiments of the invention, the apex A of each field of view F is located at the photosensitive surface of the corresponding light sensor 1. For enough sensitivity to the eye direction of the measured light intensity value which is outputted by each light sensor 1 that is oriented towards the user's eye, at least 30% of the field of view F of this light sensor, preferably at least 50%, is occupied by the wearer's eye.

In alternative embodiments, an optical fiber segment may be used between the photosensitive surface of each light sensor 1 and the corresponding field of view F. Then, the aperture value and the shape of the field of view F may be determined by the type of the optical fiber used, in particular its f-number value. Such embodiments allow placing the light sensors 1 in the frame 11 at distance from the see-through area concerned L or R and optically coupling the light sensors to the desired locations for the apex A of the fields of view F via the optical fiber segments.

It is also possible arranging a semi-reflecting holographic layer across part or all of the see-through area R or L for re-directing through reflection the fields of view F of the light sensors 1 towards the wearer's face. Such embodiments allow locating the light sensors 1 within recesses of the frame 11 around the see-through areas for aesthetic issues. The semi-reflecting holographic layer may be efficient in a narrow wavelength range for the light impinging on it, for example limitedly in a wavelength range of 4 nm (nanometer) to 10 nm width. This avoids that the holographic layer disturbs the wearer's color vision. Then, the light sensors 1 which are oriented towards the wearer's eye through reflection by the holographic layer may be preferably combined with a spectral optical filter that matches the holographic layer efficiency range.

Preferably, the whole information captured by the light sensors 1 has low redundancy. To this end, it is preferred that the axial directions D of all fields of view F do not converge to a single convergence point located in the wearer's eye, otherwise the symmetry of the iris would produce information redundancy. Hence, the fields of view F may be oriented so that their axial directions D exhibit at least two convergence points that are each common to at least two fields of view F, or so that at least some of the axial directions D do not cross any other one. For similar reason, overlaps between the fields of view F at the wearer's face are preferably low, in particular such that the sum of aperture values of field-of-view portions that are each common to at least two of the fields of view F is less than 40%, preferably less than 10%, of the sum of respective aperture values of all fields of view F.

In improved embodiments of the invention, the fields of view F may be associated in pairs having same axial direction D and substantially same field-of-view apex A, but with different aperture values within each pair, for example conical fields of view with angle values 2·θ equal to 10° and 40° within each pair (see Figure 2b). Such configuration of the fields of view provides more reliable detection information even when ambient light varies. Such improved embodiments may be obtained by distributing the light sensors 1 in pairs, with both light sensors of one and same pair that are close to one another. Alternatively, each pair of fields of view may be obtained by combining one light sensor with an active device suitable for varying the aperture value of the field of view. Such active device may be a variable focal length lens, of any type including variable membrane lenses or a liquid crystal lenses or variable refractive index lenses as examples. Then, the eyewear 10 comprises a controller for varying the aperture value quickly between two successive light intensity measurements, for example a first measurement with the angle value of 10° and a second measurement with the angle value of 40° performed immediately after the first measurement. Figure 2b illustrates such operation, in which F₁ and F₂ denote both fields of view with same axial direction D and apex A but respective angle values of 10° and 40°.

The eyewear 10 may further comprise additional light sensors that are dedicated to assessing a light map of the wearer's surroundings or of the wearer's face. The fields of view of such additional light sensors are oriented away from the wearer's eye, for example away from the wearer's face or towards a portion of the wearer's face skin. The additional light sensors provide additional measured light intensity values which may be useful for separating contributions to the measured light intensity values that relate to the eye direction from other contributions due to the wearer's surroundings and to variations related to head movements of the wearer. Possibly, an inertial measurement unit (not represented) may also be accommodated in the frame 11 for sensing the head movements and allowing subtraction of contributions of the head movements in variations of the measured light intensity values which are outputted by those of the light sensors that are directed towards the wearer's eye.

Possibly, those of the additional light sensors that are oriented away from the wearer's face may be designed for assessing polarization features of some light existing in the wearer's surroundings. This may be useful for example if the wearer is close to or in the vicinity of a water surface. For example, two of these additional light sensors may have identical fields of view, but one without polarization filtering capability and the other one provided with a polarization filter. Such design for at least some of the additional light sensors may be useful for taking into account effects of significantly polarized light that is impinging onto the wearer's eye.

All light sensors 1 used in the eyewear 10 are preferably sensitive in the visible range, i.e. from 360 nm (nanometer) to 780 nm, and possibly sensitive to a limited part within the visible range. Therefore, suitable spectral filters may be combined with the light sensors 1, preferably filters that are identical for all these light sensors. It may be advantageous that those of the light sensors 1 that are oriented towards the wearer's face - skin portion or the eye - are arranged so that light received by these sensors does not pass through an eyeglass that is situated in the see-through area L or R when propagating between the wearer's face and the light sensor. These light sensors may thus be located outside the eyeglass in front of the posterior face of the eyeglass, close to its peripheral edge. However, for the additional light sensors which are oriented away from the wearer's face, they are preferably located so that light received by these additional sensors passes through the eyeglass for automatically taking into account modifications in the lightening of the wearer's face that are produced by the eyeglass. This is helpful in particular when the eyeglass is light-absorbing, including a photochromic eyeglass, polarization-filtering or has optical power. Such arrangements for all the light sensors further improve the reliability of the determination of the eye direction.

Preferably again, the light sensors 1 of the eyewear 10 may be combined with at least one filter suitable to discard contributions to the measured light intensity values that are associated with time-variations having frequencies equal to 50 Hz (hertz), 60 Hz, 100 Hz or 120 Hz. Such time-filtering avoids determining erroneously variations in the eye direction when the ambient light is artificial one. If artificial light with time-variations corresponding to one of the above-mentioned frequency values is detected, the light sensors may be controlled for performing their respective light intensity measurements simultaneously, so that the ambient light time-variations do not interfere with the actual eye direction in the measurements.

Each eye direction is determined from a set of measured light intensity values which are captured by the light sensors 1 simultaneously and are transmitted by these latter to a processing unit 4 (see Figure 1). The processing unit 4 may be located within the frame 11, for example within one of the temples 12 and 13. A power source 3, for example an electrical battery, may also be accommodated in the frame 11 for supplying the processing unit 4 with necessary energy. The power source 3 may also be used for applying suitable bias voltage to the light sensors 1.

For increasing reliability of the determined eye direction, it is preferred to perform a pre-processing of the measured light intensity values outputted by those of the light sensors that are oriented towards the wearer's eye. Such pre-processing aims at correcting the measured light intensity values for effects of the surrounding light which may be reflected on the wearer's eye differently for one of the fields of view F compared to another one.

A first possible pre-processing only implements light sensors that are oriented towards the wearer's eye. For suppressing the effect of a general ambient light level, the measured light intensity values that are outputted by the light sensors may be transformed into normalized deviation values in the following manner. First, a mean value is computed for the measured light intensity values, then this mean value is subtracted to each of the measured light intensity values, and each subtraction result is further divided by the mean value. In this way, normalized deviation values are obtained which suppress the effect of the ambient light level on the further-processed values.

Second possibilities for pre-processing the measured light intensity values implement those of the light sensors 1 - previously called additional light sensors - that are oriented towards the face skin of the wearer outside his eye, for example towards skin portions located on the left and right sides of the wearer's face. Then, the measured light intensity value that is outputted by one of the light sensors 1 oriented towards the wearer's eye may be reduced by an amount which depends mainly on the measured values outputted by the additional sensors having parallel axial directions D but oriented towards skin portions. All the measured values outputted by the additional light sensors 1 may also be taken into account for correcting the measured light intensity value that is outputted by one of the light sensors 1 oriented towards the wearer's eye, but major role is preferably assigned to those corresponding to axial directions D that have small angular differences with the eye-oriented light sensor considered.

A third possible pre-processing implement pairs of fields of view as illustrated in Figure 2b. Each light sensor 1 performs a first measurement with field of view F₁, and immediately thereafter a second measurement with field of view F₂. Both measurements are preferably performed quickly after one another so that they correspond to same eye direction. Then, a difference is computed for each light sensor 1 between the first and second measurement, and the difference result constitutes the pre-processed values.

Fourth possible pre-processings implement additional light sensors that are oriented away from the wearer's face. For example, three to eight additional light sensors are dedicated to measure ambient light in different directions so as to assess a light map of the wearer's surroundings. Such light map allows inferring lighting spatial variations that affect the wearer's eye differently in each field of view, and then correcting for such spatial variations the measured light intensity values which are outputted by those of the light sensors that are oriented towards the wearer's eye, in a way similar to the second possibilities above. According to an improvement for assessing the light map more accurately, the invention eyewear may further accommodate an inertial measurement unit which allows separation of the ambient light spatial variations from the movements for the wearer's head.

Also possibly, several of the previous pre-processing modes may be combined for obtaining even more reliable eye direction determination. The eye direction is then determined from the pre-processed measured light intensity values.

Alternatively, it is possible not to implement any pre-processing onto some or all of the measured light intensity values and to determine the eye direction by inputting these measured light intensity values as produced by the corresponding light sensors into the algorithm for determining the eye direction.

Several algorithms may be used alternatively for determining the eye direction from the measured light intensity values, or from the pre-processed measured light intensity values. Such algorithms are known from the Man skilled in the art, so mentioning here some of them without describing them in detail is enough. Some of these algorithms are of regression type, in particular linear or polynomial regression, support vector regression, or of neural network type. Other algorithms such as nearest neighbor method, gaussian process, correlation method and 3D-modelling of the wearer's face and/or of the sensor arrangement may be used alternatively. Hybrid methods may also be used. In the present description, this step of determining the eye direction from the light intensity values, either as-measured or pre-processed, has been called processing step.

For operation of the eyewear in determining the eye direction, the processing unit may require carrying out a calibration step beforehand. Such calibration step may be useful for setting one or several parameters of the algorithm which infers the eye direction from the measured light intensity values. In particular, such calibration step may consist in acquiring labeled training sets of measured light intensity values and associating them with specified eye directions. Such calibration step may relate to general parameters that are independent from the wearer, such as the type of the eyewear, sensor calibration and locations, etc., but also parameters that relate to the wearer. Such latter wearer-depending parameters may concern eye color, skin color, face shape, ophthalmic correction, eyeglass type, wearer's position when reading a paper or climbing stairs, usual head movements, usual position of the eyewear on the wearer's face, etc. Possibly, a general training step may be implemented first, using a training dataset relating to several wearers, and thereafter an additional training step that relates individually to the wearer considered. The additional training step consists in adjusting parameters of the eye direction determination algorithm especially for the wearer, using a method which may be either the same as that for the general training step or a different one. For example, when this method is different from that of the general training step, the additional training step may consist in rescaling light intensity values and eye directions for the wearer. It is also possible in the additional training step to capture measured light intensity values only when the wearer is performing identified eye movements, and using these captured values to adjust some parameters of the algorithm implemented for eye direction determination. In such case, the additional training step may not use any training dataset.

When the eyewear is likely to fit on the user's face according to several positions, for example spectacles that may be located at two positions along the user's nose, it may be advantageous to provide it with two sets of light sensors, each appropriate for one of the wearing positions. In particular, the orientations of the fields of view may depend on the wearing position, for example through the separating distance between the light sensors and the user's eye. Thus, a first set of light sensors may be dedicated to the wearing position close to the user's face, and a second set of light sensors may be dedicated to the wearing position further from the user's face. Possibly, both light sensor sets may have at least one common light sensor whereas other ones are dedicated to only one of the sets. Selecting automatically one of the light sensor sets for determining the eye direction, based on at least some of the measured light intensity values, may also result from special aspects of the calibration step. Such selection of the light sensor set to be used may be replaced by a variable weighting that is applied by the processing unit onto all the measured light intensity values depending on the wearing position. Also possibly, the processing step can first estimate the eyewear position and secondly apply a mathematical model depending on the eyewear position. The mathematical model is then different for each eyewear position but uses all light sensors. The model used may induce less impact for some of the sensors compared to others.

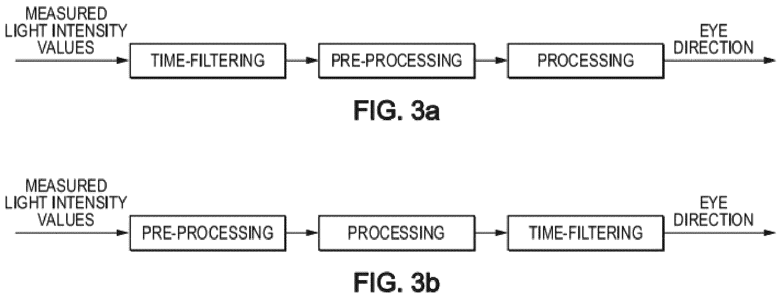

Further improvements in the reliability of the eye direction determined by the invention eyewear may be obtained by taking into account that the time-variations in the eye direction are continuous. To this end, a time-filtering, for example of Kalman-type, may be implemented either on the measured light intensity values which are outputted successively, or on eye directions which are determined successively. Figures 3a and 3b relate to such improvements, with the time-filtering performed on the measured light intensity values before the pre-processing step (Figure 3a), or after the processing step (Figure 3b). Alternatively, the time-filtering may be performed on the pre-processed measured light intensity values but before the processing step, or in combination with the processing step. Implementing a Kalman filter of predicting type may be useful when no eye direction can be determined actually, such actual determination being then replaced by the Kalman-predicted eye direction.

Knowledge of the instant eye direction is useful for many applications including protection against dazzling, polarization filtering, for instance polarization filtering limited to eye directions oriented downwardly, optical power modulation depending on focusing distance, augmented reality, informative eyewear or mixed reality and virtual reality eyewear, etc. For informative eyewear, the information is often not mobile but could be displayed only if or when the wearer is looking through a specific part of the lens. Depending on each application, the eyewear may have several forms including spectacles, goggles, a mask, for example a diving or skiing mask, a helmet, etc. Then, the eye direction may be used for controlling various functions such as electrochromic eyeglass darkening, polarization filter control, focus length modulation, display location, etc.

The light sensors may be affected by measurement disturbances such as a variation in light conditions or a slippage of the eyewear. Indeed, when the eyewear according to the invention slips, the point of view of the light sensors changes. Also, the amount of light perceived by the light sensors depends on the ambient light conditions, which may vary in time. Other possible measurement disturbances may include variation of the pupil size, movement of the eyelid, make-up, tired or wounded eye, eyelash flexibility, and the like. Measurement disturbances are generically called, in this document, measurement context, or more simply context. The context influences the way that the measurements by the light sensors of the main system vary according to the movements of the user's eye. Such variations may affect correlations between the measurements from the light sensors and the user's eye direction.

Additional robustness to light variation, to eyewear slippage, and more generally to measurement disturbances may be provided by a corrective system.

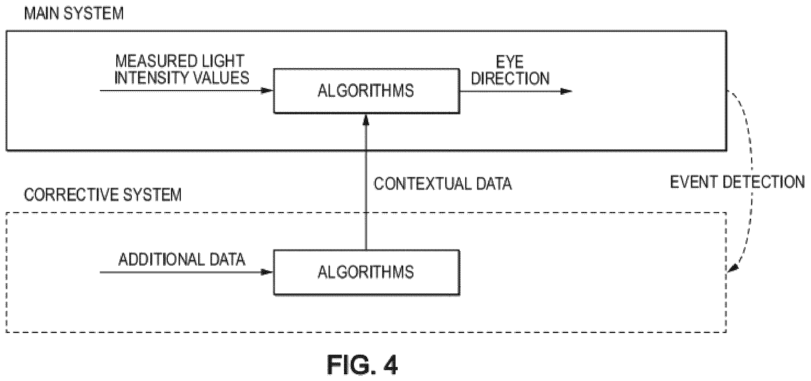

Figures 4 to 6 relate to such improvements, with an eyewear comprising a main system that comprises the at least three light sensors and the processing unit as described above, and that may be ran, for instance, at a desired frequency noted f1. The main system obtains as inputs measured light intensity values and determines as an output an eye direction of a user's eye, for instance according to Figure 3a or Figure 3b.

As depicted on Figure 4, the eyewear may further comprise a corrective system configured to provide the main system with contextual data, which allows a more robust estimation by the main system of the direction of the user's eye. To do so, the corrective system comprises one or more additional sensors arranged in the eyewear. Such additional sensors are suitable for sensing additional data relating to context. The corrective system further comprises a processing module that receives the sensed additional data from the one or more additional sensors. The processing module determines contextual data based on the sensed additional data and provides the contextual data to the processing unit of the main system. The main system is further configured to receive the contextual data and to estimate the eye direction of the user's eye further according to the contextual data.

When a measurement disturbance is identified by the corrective system, the contextual data transmitted to the processing unit of the main system are indicative of the identified measurement disturbance. The processing unit may then decorrelate the measured light intensity values from the context. In other words, the processing unit may be configured to determine the eye direction of the user's eye by choosing a correct mathematical model depending on the context within a set of models, or by considering a model which uses context as input in addition to light measurements, or even by decorrelating disturbance from data before applying the model.

In a simple example, the corrective system may comprise an inertial measurement unit, being a known combination of sensors including accelerometers, gyroscopes and magnetometers. According to known methods, a movement of the eyewear or a head movement of the user wearing the eyewear may be detected and optionally characterized based on the combination of data sensed by the sensors of the inertial movement unit. Therefore, the processing module of the corrective system may provide to the processing unit of the main system, as contextual data, an indication that a movement of the eyewear or of the head of the user has been detected, or an identifier of a type, a direction, a magnitude, a speed, etc. of such movement.

In another example, the corrective system may comprise, as an additional sensor, a small, low power and low-resolution camera or array or matrix of photodetectors, that is arranged to be facing an eye of the user when the eyewear is normally worn by the user. By processing an image output by such additional sensor, the processing module of the corrective system may:
- deduce the position of the eyewear relatively to the user's face and detect eyewear slippage, and/or
- determine a lightmap indicating how the user face is lit up and detect a level of ambient light, and/or
- determine contextual data relating to other context elements, and/or
- estimate, independently from the main system, a direction of the eye of the user that is faced by such additional sensor.

In yet another example, the corrective system may be configured to detect eye blinks of an eye of the user. There are various known ways of detecting blinks based on inputs from sensors. Optionally, the corrective system may comprise one or more dedicated sensors for detecting such eye blinks. Alternatively, or in combination, eye blinks may be detected by processing the measured light intensity values output by the light sensors of the main system, possibly in real-time. During a blink, since the eye is closed, any measured light intensity values output by the light sensors only depend on context such as eyewear position or light environment and do not depend on eye orientation. The corrective system may then run an algorithm that can estimate information about the context using these measurements during eye blinks. As the user's eyes blinks regularly, the context may be updated regularly at each blink by the corrective system, and may be provided regularly to the main system in order to improve the robustness of the determination of the user's eye direction.

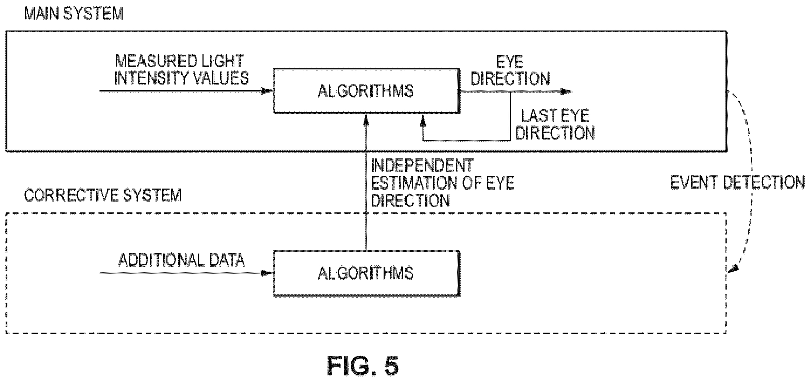

Some of the possible algorithms that may be implemented at the processing unit of the main system tend to generate a bias in the estimation of the eye direction of a user's eye. As depicted on Figure 5, the corrective system may help reducing this bias by performing an independent estimation of the eye direction of the user's eye regularly and by providing the independent estimation to the processing unit of the main system.

For instance, the processing unit may be devoid of a tracking functionality. In such a case, a single estimation of the eye direction of a user's eye is performed by the processing unit of the main system per each new set of measurements received at a given time from the light sensors, without taking into account any temporal or dynamical effects. An independent estimation of the eye direction of the user's eye provided by the corrective system may be helpful to confirm consistent estimations by the main system and/or to dismiss any inconsistent or outlier estimations by the main system.

Alternatively, the processing unit of the main system may include a tracking functionality, based for instance on a Kalman filter, on a recurrent neural network or on any equivalent algorithm. Due to taking into account temporal or dynamical effects, a tracking functionality is prone to drifts, since an erroneous estimation of the eye direction of a user's eye at a given instant would also be used to estimate the eye direction of the user's eye at subsequent instants. An independent estimation of the eye direction of the user's eye provided by the corrective system may be helpful to recalibrate the main system and avoid such drifts.

To do so, the corrective system may comprise a standalone eye-tracker, in the sense of an eye-tracker that is separate from the main system. The standalone eye-tracker may be any known device capable of determining the eyeball direction based on inputs from sensors, and relying on a different technology than the combination of an arrangement of light sensors and of a processing unit according to the main system. The standalone eye-tracker comprises, at least, one or more additional sensors suitable to sense additional data related to the eye direction of the user's eye and a processing module configured to receive the additional data from the additional sensors, determine the independent estimation of the eye direction based on the sensed additional data, and provide the independent estimation to the processing unit of the main system.

In an example, the standalone eye-tracker may comprise one or more infrared light sources, such as LED, arranged to illuminate an eye of the wearer, a set of infrared photodetectors arranged to sense the infrared light reflected by the eye of the wearer, and a processing module configured to collect the outputs of the set of infrared photodetectors. Based on a mathematical model linking infrared light measurements and eye direction, the processing module can determine eye direction from infrared lights measurements and then deduce the resulting disturbance within the visible light measurement. By knowing this disturbance, it is possible to decorrelate visible light measurements from light disturbance or to introduce this disturbance within the mathematical model to adapt the model to this new lighting condition. Optionally, the measuring step of said one or more infrared light sources may be modulated to allow performing synchronous detection with the light sensors of the main system, in order to remove disturbance due to light variation at lower frequencies than the chosen modulation.

In yet another example, the standalone eye-tracker may comprise one or several interferometer sensors based on laser feedback interferometry. From such measurement sensors, it is possible to deduce the observed target velocity, the distance between the sensor and the target, the light phase and the light polarization. Those data may be processed by a processing module to determine the eye velocity, the eye position relatively to glasses and the user line of sight. Optionally, these sensors may be combined with a 2D rotating micro-mirror (MEMS) to scan the eye region to obtain a signal which can be presented as a 2D image of the eye.

In yet another example, the standalone eye-tracker may comprise an infrared laser with a 2D rotating micro-mirror (MEMS) to scan the eye region, and with one or more photodetectors measure the laser reflection on the user's eye. As shown by recent studies, this system enables to generate an image of the eye region, which may be processed to deduce for instance eyewear slippage and/or eye direction of the user's eye.

Both functionalities of providing contextual data as depicted on Figure 4 and providing an independent estimation of the eye direction of a user's eye as depicted on Figure 5 are not mutually exclusive. In other words, the corrective system may comprise all the required additional sensors to provide not only an independent estimation of the eye direction of a user's eye, but also contextual data.

Some of the possible examples of corrective systems described above may be more consuming than the main system due to an embedded light or a power consuming component (camera, MEMS). In addition, there is a remaining uncertainty on health security regarding the use of incoherent infrared light source or laser directed to the eye and the retina for whole days (more than 8 hours) and for several years.

Regarding these two reasons, it is suggested to use the corrective system only at a low frequency or during a short time frame following a given event detection. In an embodiment, the corrective system is ran at a frequency that is different from the desired frequency f1 that the main system is ran at. For instance, the corrective system may be configured to obtain, from the one or more additional sensors, the additional data at a frequency f2 lower than f1. The lower frequency f2 allows minimizing energy consumption by the corrective system. With this configuration, the corrective system may automatically and periodically monitor any possible occurrences of measurement disturbances during normal operation of the main system, and may automatically and periodically monitor the eye direction of a user's eye in parallel of the main system.

Instead or in addition, the corrective system may have an inactive, or standby, mode and at least one active mode that is activated only temporarily, for instance in response to a detection of an event. The corrective system only provides the additional data to the main system when in the active mode. The inactive or standby mode contributes to minimizing energy consumption by the corrective system. Event detection may be performed at the processing unit and may be based, for example, on a sudden variation in measurements obtained over time from a given available sensor (such as a light sensor of the main system), and/or on a detection of a head movement by an inertial measurement unit arranged in the eyewear, and/or on a low level of trust, as assessed by an artificial intelligence based on the available data output by the available sensors, in the eye direction estimation provided by the processing unit. Event detection may be performed by comparing the data received as inputs by the processing unit and/or the data provided as outputs by the processing unit to various data patterns that may be associated to corresponding types of events in an event database.

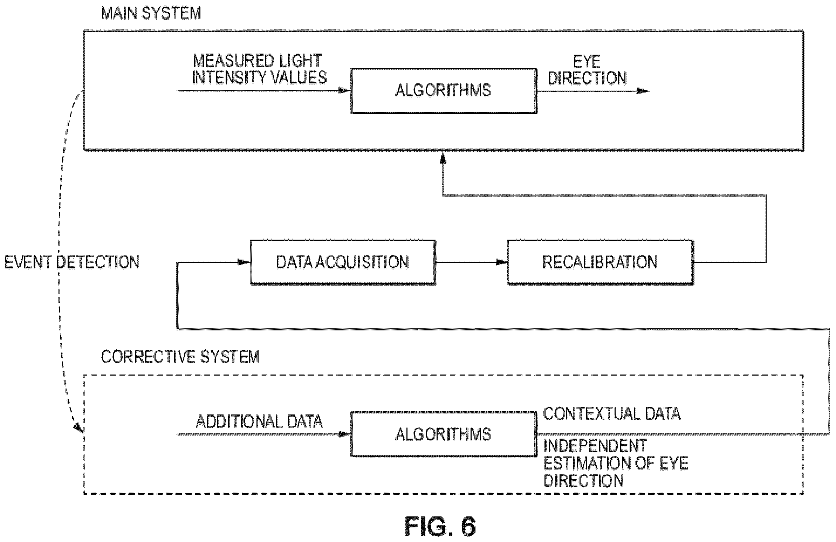

The corrective system may also be advantageous for an initialization or a recalibration of the main system, as depicted on Figure 6.

A recalibration procedure of the main system may be conducted periodically or be triggered by an event. An example of criterion defining such an event is when the pertinence, or estimated level of trust associated by an artificial intelligence to the estimation of the eye direction provided by the main system remains below a given threshold even after contextual data have already been recently received from the corrective system and processed by the main system. However, the recalibration procedure should not be applied too frequently to avoid a high-energy consumption.

Such a recalibration procedure involves temporarily activating the corrective system. An initial activation of the main system may also be interpreted as an example of event leading to a temporary activation of one or more functionalities of the correction system as part of an initialization procedure.

To perform an initialization or recalibration procedure of the main system, it is necessary to acquire a substantial amount of additional data, which may comprise raw sensor measurements, contextual data such as an eyewear position or orientation or a lightmap of a user's eye and/or an independent estimation of the eye direction of the user's eye. This additional data may be furnished by the corrective system and its embedded sensors. In order to minimize the time length of the initialization or recalibration procedure, it is desirable to quickly acquire a sufficient quantity of data.

The corrective system may then be ran at a frequency f3 higher than f1 during a narrow time interval corresponding to the acquisition of the additional data required for the initialization or recalibration algorithm to select, determine or update a mathematical model used by the processing unit of the main system. Said update may include for instance optimizing the mathematical model for a current context based on the contextual data. Said update may further rely on the independent estimation of the eye direction, for example by using said independent estimation as a criterion for selecting or determining the mathematical model, and/or as an offset in a predetermined or preselected mathematical function of the mathematical model, and/or as a coefficient or as a weighted value in a predetermined or preselected mathematical model.

The frequency higher than f1 may correspond to a dedicated active mode, namely an accelerated acquisition mode. After the additional data required for the continuation of the initialization or recalibration procedures is obtained, the frequency of the data acquisition by the additional sensors of the corrective system may be reduced by automatically switching the corrective system to another mode, which may be another active mode or the inactive or standby mode.

During the initialization or recalibration procedure, the main system may continue running in parallel, based on a default or pre-update mathematical model.

## Claims

1. An eyewear (10) adapted for fitting a user's face and for determining an eye direction of a user's eye, said eyewear comprising:
- at least three light sensors (1) arranged in the eyewear (10) for said user's eye, each of said light sensors being configured for outputting at least one measured light intensity value that corresponds to ambient light originating from surroundings of the user outside the eyewear and reflected or scattered by the user's face within a field of view (F) effective for said light sensor; and
- a processing unit (4) arranged for receiving the measured light intensity values and for determining the eye direction from said measured light intensity values,
wherein the light sensors (1) are arranged so that, when the eyewear (10) is worn by the user:
for each of the light sensors (1), the field of view (F) effective for said light sensor has an apex (A) at the eyewear (10) and an axial direction (D) oriented towards the user's face, and a cross-section increasing continuously along the axial direction from the eyewear towards the user's face,
each field of view (F) contains at least part of the user's eye,
**characterized in that**
the light sensors (1) are arranged so that, when the eyewear (10) is worn by the user, each field of view (F) has an integrated aperture value comprised between 0.006 steradian and 0.22 steradian, and the user's eye occupies at least 30% of said field of view.

2. The eyewear (10) of claim 1, wherein each light sensor (1) comprises a photodiode, a phototransistor, an ambient light sensor, or a photovoltaic cell.

3. The eyewear (10) of claim 1 or 2, wherein the respective fields of view (F) of the at least three of the light sensors (1) have conical shapes with circular cross-sections, and aperture angles (2·θ) comprised between 5° and 30°, preferably between 8° and 15°.

4. The eyewear (10) of any one of the preceding claims, comprising a see-through area (L, R) dedicated to the user's eye when the eyewear is worn by the user,
and wherein each light sensor (1) is located near a peripheral edge of the see-through area (L, R), or light from the user's face is reflected towards the light sensors by a holographic mirror that extends across the see-through area.

5. The eyewear (10) of any one of the preceding claims, comprising at least one of the following components for determining the field of view (F) of each light sensor (1):
- a lens with fixed focal length coupled to the light sensor (1);
- a variable focal lens coupled to the light sensor (1), said variable focal lens having a focal length value adjusted by a controller of the eyewear (10);
- an optical fiber segment coupled to the light sensor (1); and
- a hole with slanted peripheral wall or an aperture stop (2) arranged above a photosensitive surface of the light sensor (1).

6. The eyewear (10) of any one of the preceding claims, wherein respective axial directions (D) of a first subset of the fields of view (F) all pass through one first common convergence point located within the user's eye, and another subset of the fields of view all pass through one second common convergence point also located within the user's eye, said first and second common convergence points being located at different depth values within the user's eye when the eyewear is worn by the user.

7. The eyewear (10) of any one of the preceding claims, wherein the light sensors (1) are arranged so that a ratio of a sum of aperture values of field-of-view portions that are each common to at least two of the fields of view (F), over a sum of respective aperture values of all the fields of view, is less than 40%, preferably less than 10%.

8. The eyewear (10) of any one of the preceding claims, wherein the processing unit (4) is configured for calculating a normalized deviation value from each measured light intensity value, said normalized deviation value equaling a result of a difference between the measured light intensity value and a mean value of the measured light intensity values used for one and same determination of the eye direction, divided by said mean value,
and the processing unit (4) is further configured for determining the eye direction from the normalized deviation values.

9. The eyewear (10) of any one of the preceding claims, wherein the light sensors (1) are arranged so as to determine pairs of fields of view (F₁, F₂), with each pair containing two fields of view having common axial direction (D) and apex (A) but different aperture values,
and wherein the processing unit (4) is configured to calculate for each pair a combination between the respective light intensity values measured for both fields of view (F₁, F₂) of said pair, and to determine the eye direction from results of said differences related to at least three pairs of fields of view.

10. The eyewear (10) of any one of the preceding claims, further comprising additional light sensors with respective fields of view oriented away from the user's eye when the eyewear is worn by the user, and dedicated to assessing a light map of the user's surroundings or of the user's face,
and wherein the processing unit (4) is configured to determine the eye direction from the measured light intensity values relating to the light reflected or scattered by the user's eye in combination with the light map as assessed by the additional light sensors.

11. The eyewear (10) of any one of the preceding claims, wherein the processing unit (4) is configured to determine the eye direction using one of the following algorithm types:
- regression type, in particular linear and polynomial regression, support vector regression, neural network type;
- nearest neighbor method, gaussian process and correlation method; and
- an algorithm based on a 3D-model of the user's face and/or of the light sensor arrangement in the eyewear (10).

12. The eyewear (10) of any one of the preceding claims, configured to output successive sets of measured light intensity values, each set corresponding to a respective eye direction determination sequence,
and wherein the processing unit (4) is configured to determine successive filtered eye directions by implementing a time-filter, in particular a Kalman filter, either with the successive sets of measured light intensity values, or with successive eye directions as determined each separately from one of the successive sets of measured light intensity values.

13. The eyewear (10) of any one of the preceding claims, configured to perform a calibration step prior to useful operation for determining the eye direction, the calibration step consisting in acquiring labeled training sets which are each comprised of measured light intensity values and a corresponding eye direction, and adjusting parameters of an algorithm that is implemented by the processing unit (4) for the useful operation of the eyewear for determining the eye direction, based on the labeled training sets.

14. The eyewear (10) of any one of the preceding claims, of one among the following types:
- spectacles provided with electrochromic lenses, and adapted for adjusting a transmission of each lens based on the eye direction determined for the corresponding user's eye, and optionally also based on a sensed direction of a light source in the user's surroundings;
- a mask provided with an electrochromic glass, and adapted for adjusting an absorption of the glass based on the eye direction determined for at least one of the user's eyes, and optionally also based on a sensed direction of a light source in the user's surroundings;
- an augmented reality eyewear, an informative eyewear or a mixed reality and virtual reality eyewear;
- an eyewear provided with variable focal lenses, and adapted for adjusting a focal length of each lens based on the eye direction determined for the corresponding user's eye; and
- spectacles or mask provided with at least one active filter and adapted for adjusting a spectral transmission or a polarization filtering operation of said filter based on the eye direction determined for at least one of the user's eyes.

15. The eyewear (10) of any one of the preceding claims, comprising two sets each of at least three light sensors (1), each set having fields of view (F) oriented so that said set is efficient for determining the eye direction for a position of the eyewear on the user's face, the respective eyewear positions that correspond separately to all light sensor sets being different from each other.

16. The eyewear (10) of any one of the preceding claims, comprising a corrective system configured to provide, to the processing unit, contextual data relating to a context of measured light intensity values by the at least three light sensors for a given determination of the eye direction, and the processing unit being configured to determine the eye direction further based on the contextual data.

17. The eyewear (10) of claim 16, wherein the corrective system comprises at least one additional sensor and the contextual data are based on an output of the at least one additional sensor.

18. The eyewear (10) of claim 16 or 17, wherein the corrective system comprises a standalone device for sensing additional data relating to the eye direction of the user's eye, the corrective system being configured to determine an independent estimation of the eye direction based on an output of the standalone device and to provide the independent estimation to the processing unit.

19. The eyewear of any one of claims 16 to 18, wherein the at least three sensors are configured for outputting the measured light intensity values at a given frequency and the at least one additional sensor is configured for outputting the additional data at a frequency different from the given frequency.

20. The eyewear any one of claims 16 to 19, wherein the corrective system comprises an activation module for temporarily switching the corrective system from an inactive mode to an active mode upon detection of an event, said detection being based on an output of at least one sensor.

## Patentansprüche

1. Brille (10), die zum Passen auf ein Gesicht eines Benutzers und zum Bestimmen einer Blickrichtung eines Auges eines Benutzers eingerichtet ist, wobei die Brille Folgendes umfasst:
- wenigstens drei Lichtsensoren (1), die in der Brille (10) für das Auge des Benutzers angeordnet sind, wobei jeder der Lichtsensoren dazu konfiguriert ist, wenigstens einen gemessenen Lichtintensitätswert auszugeben, der Umgebungslicht entspricht, das aus einer Umgebung des Benutzers außerhalb der Brille stammt und durch das Gesicht des Benutzers innerhalb eines für den Lichtsensor wirksamen Sichtfeldes (F) reflektiert oder gestreut wird; und
- eine Verarbeitungseinheit (4), die zum Empfangen der gemessenen Lichtintensitätswerte und zum Bestimmen der Blickrichtung aus den gemessenen Lichtintensitätswerten eingerichtet ist,
wobei die Lichtsensoren (1) so angeordnet sind, dass, wenn die Brille (10) durch den Benutzer getragen wird:
für jeden der Lichtsensoren (1), das für den Lichtsensor wirksame Sichtfeld (F) einen Scheitelpunkt (A) bei der Brille (10) und eine axiale Richtung (D), die zu dem Gesicht des Benutzers hin orientiert ist, und einen Querschnitt, der entlang der axialen Richtung von der Brille zu dem Gesicht des Benutzers hin kontinuierlich zunimmt, aufweist,
jedes Sichtfeld (F) wenigstens einen Teil des Auges des Benutzers enthält,
**dadurch gekennzeichnet, dass**
die Lichtsensoren (1) so angeordnet sind, dass, wenn die Brille (10) durch den Benutzer getragen wird, jedes Sichtfeld (F) einen integrierten Aperturwert aufweist, der zwischen 0,006 Steradiant und 0,22 Steradiant liegt, und das Auge des Benutzers wenigstens 30 % des Sichtfelds einnimmt.

2. Brille (10) nach Anspruch 1, wobei jeder Lichtsensor (1) eine Fotodiode, einen Fototransistor, einen Umgebungslichtsensor oder eine Fotovoltaikzelle umfasst.

3. Brille (10) nach Anspruch 1 oder 2, wobei die jeweiligen Sichtfelder (F) der wenigstens drei der Lichtsensoren (1) konische Formen mit kreisförmigen Querschnitten und Aperturwinkel (2·θ), zwischen 5° und 30°, vorzugsweise zwischen 8° und 15° liegen, aufweisen.

4. Brille (10) nach einem der vorhergehenden Ansprüche, die einen Durchsichtbereich (L, R) umfasst, der dem Auge des Benutzers zugeordnet ist, wenn die Brille durch den Benutzer getragen wird,
und wobei sich jeder Lichtsensor (1) in der Nähe eines peripheren Randes des Durchsichtbereichs (L, R) befindet oder Licht von dem Gesicht des Benutzers durch einen holografischen Spiegel, der sich über den Durchsichtbereich hinweg erstreckt, zu den Lichtsensoren reflektiert wird.

5. Brille (10) nach einem der vorhergehenden Ansprüche, die wenigstens eine der folgenden Komponenten zum Bestimmen des Sichtfeldes (F) jedes Lichtsensors (1) umfasst:
- eine Linse mit fester Brennweite, die mit dem Lichtsensor (1) gekoppelt ist;
- eine mit dem Lichtsensor (1) gekoppelte Linse mit variablem Fokus, wobei die Linse mit variablem Fokus einen Brennweitenwert aufweist, der durch eine Steuerung der Brille (10) eingestellt wird;
- ein Optikfasersegment, das mit dem Lichtsensor (1) gekoppelt ist; und
- ein Loch mit abgeschrägter Peripheriewand oder eine Aperturblende (2), die oberhalb einer lichtempfindlichen Oberfläche des Lichtsensors (1) angeordnet ist.

6. Brille (10) nach einem der vorhergehenden Ansprüche, wobei jeweilige axiale Richtungen (D) einer ersten Teilmenge der Sichtfelder (F) alle durch einen ersten gemeinsamen Konvergenzpunkt hindurchgehen, der sich innerhalb des Auges des Benutzers befindet, und einer anderen Teilmenge der Sichtfelder alle durch einen zweiten gemeinsamen Konvergenzpunkt hindurchgehen, der sich auch innerhalb des Auges des Benutzers befindet, wobei sich der erste und zweite gemeinsame Konvergenzpunkt bei unterschiedlichen Tiefenwerten innerhalb des Auges des Benutzers befinden, wenn die Brille durch den Benutzer getragen wird.

7. Brille (10) nach einem der vorhergehenden Ansprüche, wobei die Lichtsensoren (1) so angeordnet sind, dass ein Verhältnis einer Summe von Aperturwerten von Sichtfeldteilen, die jeweils wenigstens zwei der Sichtfelder (F) gemein sind, zu einer Summe von jeweiligen Aperturwerten sämtlicher Sichtfelder weniger als 40 %, vorzugsweise weniger als 10 %, beträgt.

8. Brille (10) nach einem der vorhergehenden Ansprüche, wobei die Verarbeitungseinheit (4) zum Berechnen eines normierten Abweichungswertes aus jedem gemessenen Lichtintensitätswert konfiguriert ist, wobei der normierte Abweichungswert gleich einem Ergebnis einer Differenz zwischen dem gemessenen Lichtintensitätswert und einem Mittelwert der gemessenen Lichtintensitätswerte, die für ein und dieselbe Bestimmung der Blickrichtung verwendet werden, geteilt durch den Mittelwert ist,
und die Verarbeitungseinheit (4) ferner dazu konfiguriert ist, die Blickrichtung aus den normierten Abweichungswerten zu bestimmen.

9. Brille (10) nach einem der vorhergehenden Ansprüche, wobei die Lichtsensoren (1) so angeordnet sind, dass sie Paare von Sichtfeldern (F₁, F₂) bestimmen, wobei jedes Paar zwei Sichtfelder mit einer gemeinsamen axialen Richtung (D) und einem gemeinsamen Scheitelpunkt (A), aber unterschiedlichen Aperturwerten enthält,
und wobei die Verarbeitungseinheit (4) dazu konfiguriert ist, für jedes Paar eine Kombination zwischen den jeweiligen Lichtintensitätswerten, die für beide Sichtfelder (F₁, F₂) des Paars gemessen werden, zu berechnen und die Blickrichtung aus Ergebnissen der Differenzen in Bezug auf wenigstens drei Paare von Sichtfeldern zu bestimmen.

10. Brille (10) nach einem der vorhergehenden Ansprüche, die ferner zusätzliche Lichtsensoren mit jeweiligen Sichtfeldern umfasst, die von dem Auge des Benutzers weg orientiert sind, wenn die Brille durch den Benutzer getragen wird, und die dazu ausgelegt sind, eine Lichtkarte der Umgebung des Benutzers oder des Gesichts des Benutzers zu erfassen,
und wobei die Verarbeitungseinheit (4) dazu konfiguriert ist, die Blickrichtung aus den gemessenen Lichtintensitätswerten bezüglich des durch das Auge des Benutzers reflektierten oder gestreuten Lichts in Kombination mit der Lichtkarte zu bestimmen, wie durch die zusätzlichen Lichtsensoren erfasst.

11. Brille (10) nach einem der vorhergehenden Ansprüche, wobei die Verarbeitungseinheit (4) dazu konfiguriert ist, die Blickrichtung unter Verwendung von einem der folgenden Algorithmustypen zu bestimmen:
- Regressionstyp, insbesondere lineare und polynomiale Regression, Stützvektorregression, Neuronales-Netz-Typ;
- Nächster-Nachbar-Verfahren, Gauß-Prozess und Korrelationsverfahren; und
- einem Algorithmus basierend auf einem 3D-Modell des Gesichts des Benutzers und/oder der Lichtsensoranordnung in der Brille (10).

12. Brille (10) nach einem der vorhergehenden Ansprüche, die dazu konfiguriert ist, aufeinanderfolgende Sätze gemessener Lichtintensitätswerte auszugeben, wobei jeder Satz einer jeweiligen Blickrichtungsbestimmungssequenz entspricht,
und wobei die Verarbeitungseinheit (4) dazu konfiguriert ist, aufeinanderfolgende gefilterte Blickrichtungen durch Implementieren eines Zeitfilters, insbesondere eines Kalman-Filters, entweder mit den aufeinanderfolgenden Sätzen von gemessenen Lichtintensitätswerten oder mit aufeinanderfolgenden Blickrichtungen, wie jeweils separat aus einem der aufeinanderfolgenden Sätze von gemessenen Lichtintensitätswerten bestimmt, zu bestimmen.

13. Brille (10) nach einem der vorhergehenden Ansprüche, die dazu konfiguriert ist, einen Kalibrierungsschritt vor einem Nutzbetrieb zum Bestimmen der Blickrichtung durchzuführen, wobei der Kalibrierungsschritt daraus besteht, gekennzeichnete Trainingssätze zu erlangen, die jeweils aus gemessenen Lichtintensitätswerten und einer entsprechenden Blickrichtung bestehen, und Parameter eines Algorithmus, der durch die Verarbeitungseinheit (4) für den Nutzbetrieb der Brille zum Bestimmen der Blickrichtung implementiert wird, basierend auf den gekennzeichneten Trainingssätzen anzupassen.

14. Brille (10) nach einem der vorhergehenden Ansprüche mit einem unter den folgenden Typen:
- eine Sehhilfenbrille, die mit elektrochromen Linsen versehen sind und zum Anpassen einer Transmission jeder Linse basierend auf der für das entsprechende Auge des Benutzers bestimmten Blickrichtung und optional auch basierend auf einer erfassten Richtung einer Lichtquelle in der Umgebung des Benutzers eingerichtet sind;
- eine Maske, die mit einem elektrochromen Glas versehen ist und dazu eingerichtet ist, eine Absorption des Glases basierend auf der für wenigstens eines der Augen des Benutzers bestimmten Blickrichtung und optional auch basierend auf einer erfassten Richtung einer Lichtquelle in der Umgebung des Benutzers anzupassen;
- eine Augmented-Reality-Brille, eine informative Brille oder eine Mixed-Reality- und Virtual-Reality-Brille;
- eine Brille, die mit Linsen mit variabler Brennweite versehen ist und zum Anpassen einer Brennweite jeder Linse basierend auf der für das entsprechende Auge des Benutzers bestimmten Blickrichtung eingerichtet ist; und
- Sehhilfenbrille oder Maske, die mit wenigstens einem aktiven Filter versehen ist und dazu eingerichtet ist, eine spektrale Transmission oder eine Polarisationsfilteroperation des Filters basierend auf der für wenigstens eines der Augen des Benutzers bestimmten Blickrichtung anzupassen.

15. Brille (10) nach einem der vorhergehenden Ansprüche, die zwei Sätze von jeweils wenigstens drei Lichtsensoren (1) umfasst, wobei jeder Satz Sichtfelder (F) aufweist, die so orientiert sind, dass der Satz effizient zum Bestimmen der Blickrichtung für eine Position der Brille auf dem Gesicht des Benutzers ist, wobei die jeweiligen Brillenpositionen, die separat allen Lichtsensorsätzen entsprechen, voneinander verschieden sind.

16. Brille (10) nach einem der vorhergehenden Ansprüche, die ein Korrektursystem umfasst, das dazu konfiguriert ist, der Verarbeitungseinheit Kontextdaten bereitzustellen, die sich auf einen Kontext durch die wenigstens drei Lichtsensoren gemessener Lichtintensitätswerte für eine gegebene Bestimmung der Blickrichtung beziehen, und wobei die Verarbeitungseinheit dazu konfiguriert ist, die Blickrichtung ferner basierend auf den Kontextdaten zu bestimmen.

17. Brille (10) nach Anspruch 16, wobei das Korrektursystem wenigstens einen zusätzlichen Sensor umfasst und die Kontextdaten auf einer Ausgabe des wenigstens einen zusätzlichen Sensors basieren.

18. Brille (10) nach Anspruch 16 oder 17, wobei das Korrektursystem eine eigenständige Vorrichtung zum Erfassen zusätzlicher Daten in Bezug auf die Blickrichtung des Auges des Benutzers umfasst, wobei das Korrektursystem dazu konfiguriert ist, eine unabhängige Schätzung der Blickrichtung basierend auf einer Ausgabe der eigenständigen Vorrichtung zu bestimmen und die unabhängige Schätzung der Verarbeitungseinheit bereitzustellen.

19. Brille nach einem der Ansprüche 16 bis 18, wobei die wenigstens drei Sensoren zum Ausgeben der gemessenen Lichtintensitätswerte bei einer gegebenen Frequenz konfiguriert sind und der wenigstens eine zusätzliche Sensor zum Ausgeben der zusätzlichen Daten bei einer Frequenz konfiguriert ist, die sich von der gegebenen Frequenz unterscheidet.

20. Brille nach einem der Ansprüche 16 bis 19, wobei das Korrektursystem ein Aktivierungsmodul zum vorübergehenden Umschalten des Korrektursystems von einem inaktiven Modus in einen aktiven Modus umfasst, wenn ein Ereignis detektiert wird, wobei die Detektion auf einer Ausgabe von wenigstens einem Sensor basiert.

## Revendications

1. Article de lunetterie (10) adapté pour s'ajuster au visage d'un utilisateur et pour déterminer une direction d'œil d'un œil de l'utilisateur, ledit article de lunetterie comprenant :
- au moins trois capteurs de lumière (1) disposés dans l'article de lunetterie (10) pour ledit œil de l'utilisateur, chacun desdits capteurs de lumière étant conçu pour restituer au moins une valeur d'intensité lumineuse mesurée qui correspond à la lumière ambiante provenant de l'environnement de l'utilisateur à l'extérieur de l'article de lunetterie et réfléchie ou diffusée par le visage de l'utilisateur à l'intérieur d'un champ de vision (F) effectif dudit capteur de lumière ; et
- une unité de traitement (4) agencée pour recevoir les valeurs d'intensité lumineuse mesurées et pour déterminer la direction de l'œil à partir desdites valeurs d'intensité lumineuse mesurées,
dans lequel les capteurs de lumière (1) sont agencés de telle sorte que, lorsque l'article de lunetterie (10) est porté par l'utilisateur :
pour chacun des capteurs de lumière (1), le champ de vision (F) effectif dudit capteur de lumière présente un sommet (A) au niveau de l'article de lunetterie (10) et une direction axiale (D) orientée vers le visage de l'utilisateur, et une section transversale augmentant de façon continue le long de la direction axiale allant de l'article de lunetterie vers le visage de l'utilisateur, chaque champ de vision (F) contient au moins une partie de l'œil de l'utilisateur,
**caractérisé en ce que**
les capteurs de lumière (1) sont agencés de telle sorte que, lorsque l'article de lunetterie (10) est porté par l'utilisateur, chaque champ de vision (F) présente une valeur d'ouverture intégrée comprise entre 0,006 stéradian et 0,22 stéradian, et l'œil de l'utilisateur occupe au moins 30 % dudit champ de vision.

2. Article de lunetterie (10) de la revendication 1, dans lequel chaque capteur de lumière (1) comprend une photodiode, un phototransistor, un capteur de lumière ambiante, ou une cellule photovoltaïque.

3. Article de lunetterie (10) de la revendication 1 ou 2, dans lequel les champs de vision (F) respectifs des au moins trois capteurs de lumière (1) ont des formes coniques avec des sections transversales circulaires, et des angles d'ouverture (2·θ) compris entre 5° et 30°, de préférence entre 8° et 15°.

4. Article de lunetterie (10) de l'une quelconque des revendications précédentes, comprenant une zone transparente (L, R) dédiée à l'œil de l'utilisateur lorsque l'article de lunetterie est porté par l'utilisateur,
et dans lequel chaque capteur de lumière (1) est situé près d'un bord périphérique de la zone transparente (L, R), ou la lumière provenant du visage de l'utilisateur est réfléchie vers les capteurs de lumière par un miroir holographique qui s'étend en travers de la zone transparente.

5. Article de lunetterie (10) de l'une quelconque des revendications précédentes, comprenant au moins un des composants suivants pour déterminer le champ de vision (F) de chaque capteur de lumière (1) :
- une lentille à distance focale fixe couplée au capteur de lumière (1) ;
- une lentille à focale variable couplée au capteur de lumière (1), ladite lentille à focale variable présentant une valeur de distance focale ajustée par un contrôleur de l'article de lunetterie (10) ;
- un segment de fibre optique couplé au capteur de lumière (1) ; et
- un trou à paroi périphérique inclinée ou un diaphragme d'ouverture (2) disposé au-dessus d'une surface photosensible du capteur de lumière (1).

6. Article de lunetterie (10) de l'une quelconque des revendications précédentes, dans lequel des directions axiales (D) respectives d'un premier sous-ensemble des champs de vision (F) passent toutes par un premier point de convergence commun situé à l'intérieur de l'œil de l'utilisateur, et d'un autre sous-ensemble des champs de vision passent toutes par un deuxième point de convergence commun également situé à l'intérieur de l'œil de l'utilisateur, lesdits premier et deuxième points de convergence communs étant situés à différentes valeurs de profondeur à l'intérieur de l'œil de l'utilisateur lorsque l'article de lunetterie est porté par l'utilisateur.

7. Article de lunetterie (10) de l'une quelconque des revendications précédentes, dans lequel les capteurs de lumière (1) sont agencés de telle sorte qu'un rapport entre une somme de valeurs d'ouverture de parties de champ de vision qui sont chacune communes à au moins deux des champs de vision (F) et une somme de valeurs d'ouverture respectives de tous les champs de vision est inférieur à 40 %, de préférence inférieur à 10 %.

8. Article de lunetterie (10) de l'une quelconque des revendications précédentes, dans lequel l'unité de traitement (4) est conçue pour calculer une valeur d'écart normalisée à partir de chaque valeur d'intensité lumineuse mesurée, ladite valeur d'écart normalisée étant égale à un résultat d'une différence entre la valeur d'intensité lumineuse mesurée et une valeur moyenne des valeurs d'intensité lumineuse mesurées utilisées pour une seule et même détermination de la direction de l'œil divisée par ladite valeur moyenne,
et l'unité de traitement (4) est en outre conçue pour déterminer la direction de l'œil à partir des valeurs d'écart normalisées.

9. Article de lunetterie (10) de l'une quelconque des revendications précédentes, dans lequel les capteurs de lumière (1) sont agencés de manière à déterminer des paires de champs de vision (F₁, F₂), chaque paire contenant deux champs de vision ayant une direction axiale (D) et un sommet (A) communs, mais des valeurs d'ouverture différentes,
et dans lequel l'unité de traitement (4) est conçue pour calculer pour chaque paire une combinaison entre les valeurs d'intensité lumineuse respectives mesurées pour les deux champs de vision (F₁, F₂) de ladite paire, et pour déterminer la direction de l'œil à partir de résultats desdites différences associées à au moins trois paires de champs de vision.

10. Article de lunetterie (10) de l'une quelconque des revendications précédentes, comprenant en outre des capteurs de lumière supplémentaires avec des champs de vision respectifs orientés à l'opposé de l'œil de l'utilisateur lorsque l'article de lunetterie est porté par l'utilisateur, et dédiés à l'évaluation d'une carte lumineuse de l'environnement de l'utilisateur ou du visage de l'utilisateur,
et dans lequel l'unité de traitement (4) est conçue pour déterminer la direction de l'œil à partir des valeurs d'intensité lumineuse mesurées relatives à la lumière réfléchie ou diffusée par l'œil de l'utilisateur combinées à la carte lumineuse telle qu'évaluée par les capteurs de lumière supplémentaires.

11. Article de lunetterie (10) de l'une quelconque des revendications précédentes, dans lequel l'unité de traitement (4) est conçue pour déterminer la direction de l'œil au moyen d'un des types d'algorithme suivants :
- le type régression, notamment la régression linéaire et polynomiale, la régression à vecteurs de support, le type réseau neuronal ;
- la méthode du voisin le plus proche, le processus gaussien et la méthode de la corrélation ; et
- un algorithme basé sur un modèle 3D du visage de l'utilisateur et/ou de l'agencement de capteurs de lumière dans l'article de lunetterie (10).

12. Article de lunetterie (10) de l'une quelconque des revendications précédentes, conçu pour restituer des ensembles successifs de valeurs d'intensité lumineuse mesurées, chaque ensemble correspondant à une séquence de détermination de direction de l'œil respective,
et dans lequel l'unité de traitement (4) est conçue pour déterminer des directions de l'œil filtrées successives en mettant en œuvre un filtre temporel, en particulier un filtre de Kalman, soit avec les ensembles successifs de valeurs d'intensité lumineuse mesurées, soit avec des directions de l'œil successives telles que déterminées chacune séparément à partir d'un des ensembles successifs de valeurs d'intensité lumineuse mesurées.

13. Article de lunetterie (10) de l'une quelconque des revendications précédentes, conçu pour effectuer une étape d'étalonnage avant une opération utile servant à déterminer la direction de l'œil, l'étape d'étalonnage consistant à acquérir des ensembles d'entraînement étiquetés qui sont chacun composés de valeurs d'intensité lumineuse mesurées et d'une direction de l'œil correspondante, et à ajuster des paramètres d'un algorithme qui est mis en œuvre par l'unité de traitement (4) pour l'opération utile de l'article de lunetterie servant à déterminer la direction de l'œil, sur la base des ensembles d'entraînement étiquetés.

14. Article de lunetterie (10) de l'une quelconque des revendications précédentes, d'un des types suivants :
- des lunettes pourvues de lentilles électrochromes, et adaptées pour ajuster une transmission de chaque lentille sur la base de la direction de l'œil déterminée pour l'œil de l'utilisateur correspondant, et éventuellement également sur la base d'une direction détectée d'une source de lumière dans l'environnement de l'utilisateur ;
- un masque pourvu d'un verre électrochrome, et adapté pour ajuster une absorption du verre sur la base de la direction de l'œil déterminée pour au moins un des yeux de l'utilisateur, et éventuellement également sur la base d'une direction détectée d'une source de lumière dans l'environnement de l'utilisateur ;
- un article de lunetterie à réalité augmentée, un article de lunetterie informatif ou un article de lunetterie à réalité mixte et à réalité virtuelle ;
- un article de lunetterie pourvu de lentilles à focale variable, et adapté pour ajuster une distance focale de chaque lentille sur la base de la direction de l'œil déterminée pour l'œil de l'utilisateur correspondant ; et
- des lunettes ou un masque pourvus d'au moins un filtre actif et adaptés pour ajuster une transmission spectrale ou une opération de filtrage de polarisation dudit filtre sur la base de la direction de l'œil déterminée pour au moins un des yeux de l'utilisateur.

15. Article de lunetterie (10) de l'une quelconque des revendications précédentes, comprenant deux ensembles, chacun d'au moins trois capteurs de lumière (1), chaque ensemble présentant des champs de vision (F) orientés de telle sorte que ledit ensemble est efficace pour déterminer la direction de l'œil pour une position de l'article de lunetterie sur le visage de l'utilisateur, les positions respectives de l'article de lunetterie qui correspondent séparément à tous les ensembles de capteurs de lumière étant différentes les unes des autres.

16. Article de lunetterie (10) de l'une quelconque des revendications précédentes, comprenant un système correctif conçu pour fournir à l'unité de traitement des données contextuelles relatives à un contexte de valeurs d'intensité lumineuse mesurées par les au moins trois capteurs de lumière pour une détermination donnée de la direction de l'œil, et l'unité de traitement étant conçue pour déterminer la direction de l'œil également sur la base des données contextuelles.

17. Article de lunetterie (10) de la revendication 16, dans lequel le système correctif comprend au moins un capteur supplémentaire et les données contextuelles sont basées sur une sortie de l'au moins un capteur supplémentaire.

18. Article de lunetterie (10) de la revendication 16 ou 17, dans lequel le système correctif comprend un dispositif autonome destiné à détecter des données supplémentaires relatives à la direction d'œil de l'œil de l'utilisateur, le système correctif étant conçu pour déterminer une estimation indépendante de la direction de l'œil sur la base d'une sortie du dispositif autonome et pour fournir l'estimation indépendante à l'unité de traitement.

19. Article de lunetterie de l'une quelconque des revendications 16 à 18, dans lequel les au moins trois capteurs sont conçus pour restituer les valeurs d'intensité lumineuse mesurées à une fréquence donnée et l'au moins un capteur supplémentaire est conçu pour restituer les données supplémentaires à une fréquence différente de la fréquence donnée.

20. Article de lunetterie de l'une quelconque des revendications 16 à 19, dans lequel le système correctif comprend un module d'activation destiné à basculer temporairement le système correctif d'un mode inactif à un mode actif lors de la détection d'un événement, ladite détection étant basée sur une sortie d'au moins un capteur.
